# EUROPEAN PATENT SPECIFICATION

(11) **EP 1 275 712 B1**
(45) Date of publication and mention of the grant of the patent: **04.10.2006**
(21) Application number: 02017961.0
(22) Date of filing: 26.04.1996
(51) Int. Cl.: C12N 9/42, C11D 3/386

(54) **Detergents comprising cellulases**
Cellulasen enthaltende Waschmittel
Détergents comprenant des cellulases

(30) Priority: 28.04.1995 EP 95201115; 12.03.1996 US 614115
(43) Date of publication of application: 15.01.2003
(62) Divisional of application: 96914993.9
(73) Proprietor: Henkel KGaA, 40589 Düsseldorf (DE)
(72) Inventor: Lenting, Hermanus Bernardus Maria, 2641 Pijnacker (NL); Van Beckhoven, Rudolf Franciscus Wilhelmus, 4334 Breda (NL); Maurer Karl-Heinz, 40699 Erkrath (DE); Kottwitz, Beatrix, 40593 Düsseldorf (DE); Weiss, Albrecht, 40764 Langenfeld (DE); Van Solingen, Pieter, 2671 Naaldwijk (NL)

(56) References cited:
- EP-A- 0 271 004
- EP-A- 0 636 740
- WO-A-95/02675

## Description

The present invention relates to a method to provide single cellulases for detergent applications.

Cellulases, also called cellulolytic enzymes, are enzymes which are capable of the hydrolysis of the β-D-glucosidic linkages in celluloses. Cellulolytic enzymes have been divided traditionally into three classes: endoglucanases, exoglucanases or cellobio-hydrolases and β-glucosidases (Knowles, J. et al. (1987), TIBTECH 5, 255- 261). Cellulolytic enzymes can be produced by a large number of bacteria, yeasts and fungi. Microorganisms that produce cellulases are described in for example GB-A- 2094826.

Several applications have been developed for the use of cellulolytic enzymes:
- degrading (wood)cellulose pulp into sugars for (bio)ethanol production;
- several textile treatments like 'stone washing' and 'biopolishing';
- application in detergent compositions.

The use of cellulases in detergent compositions started with cellulases capable of reducing the harshness, i.e. softening, of cotton containing fabrics, as described in for example GB-B-1358599.

It is further known that detergent compositions comprising cellulases are effective in removing dirt, i.e. cleaning. The efficiency of cellulolytic enzymes, cellulases, in terms of cleaning textile has been recognized for some time. GB-A-2075028, GB-A-2095275 and GB-A-2094826 disclose detergent compositions with cellulase for improved cleaning performance.

It is also known in the art that cellulases can act as a colour clarifying agent in laundry detergents. After repeated washing of soiled fabrics, cotton containing fabrics appear to be greyish, most probably due to disrupted fibres caused by mechanical action. The fibres are torn up resulting in disordered fibres which are broken. The use of cellulases as colour clarification agents for coloured fabrics has been described in EP-A-0220016. Actually cellulase mixtures from the fungal strain Humicola insolens (DSM 1800) are commonly used in detergents to result in antipilling and colour revival properties. The cellulolytic enzyme system produced by the wild type microorganism is available under the trade name of Celluzyme® by Novo-Nordisk. In addition a cloned (single) cellulase from the same origin under the trade name Carezyme® is also used in detergents.

The main disadvantage of the cellulases known in the art showing colour clarification is that these enzymes agressively degrade the cellulose containing fabrics which results in damage by undesirable loss of tensile strength of the fabrics.

On the other hand cellulases known in the art showing good cleaning properties show hardly any colour clarification effects. The first commercial detergent with cellulases in the world contained a bacterial cellulase. This enzyme represents an above mentioned alkaline endoglucanase from a Bacillus species that does not attack cellulose fibers. The enzyme is described to give a cleaning effect during washing. No effects with respect to anti-pilling or colour revival have been described for this enzyme.

From the above it will become clear that it is still desirable to provide for improved cellulases in detergent applications. Using mixtures of cellulases, as suggested in international patent application WO-A-95/02675, is supposed to provide the above mentioned performances in laundry washing, but to our knowledge it has not previously been possible to use single enzymes providing all these characteristics when applied in laundry washing.

Surprisingly it has been found that the use of certain single cellulases which are capable of cleaning, antiredeposition, colour clarification and antipilling performance in laundry washing does not at all result in unacceptable damage to the textiles washed.

Accordingly, the present invention relates to a method to provide single cellulases for detergent applications, characterized by the steps of
(a) isolation of alkalitolerant, cellulase producing microorganisms that can be obtained from water or soil samples from an alkaline environment,
(b) purification of the cellulases from these strains,
(c) determination of tensile strength loss (TSL, as herein defined) during the washing process,
(d) determination of the antipilling properties (AP, as herein defined) during the washing process and
(e) choosing the cellulases with a ratio of tensile strength loss (TSL) to antipilling properties (AP) below 1.

The use of a single cellulase, purified from alkalitolerant, cellulase producing microorganisms that can be obtained from water or soil samples from an alkaline environment or of a derivative of that cellulase with a ratio of tensile strength loss (TSL) to antipilling properties (AP) below 1, preferably below 0.8, more particularly in the range of from 0.001 to 0.5, in aqueous laundry solutions is the subject-matter of the co-pending European application 96914993.9, publication no. EP-A-827 534.

To measure tensile strength loss is a way to measure damage caused by mechanical stress or enzymatical action on fibers. It is to be understood that for the purpose of the present invention cotton fiber has to be used. The method measures the tensile strength of single fibers under wet conditions. It is described in the German Standard DIN 53,857, part 1, as well as in the International Standard ISO 2267.

As the effect normally shows up in a significant amount only after about 20 to 25 wash cycles, there is always some tensile strength loss due to the mechanical forces acting on the cotton fiber during the washing process. Therefore the tensile strength loss of a control fabric washed without cellulases using the same formulation of detergent and the same type of washing machine and washing programme has to be subtracted. To calibrate the values, a preparation of the (single) endoglucanase V from Humicola insolens (EG V) in equal amounts of enzymatic protein in the detergent is used as a standard and the value of the tensile strength loss for this sample minus the control value of detergent without cellulase is taken as a TSL of 100%. This cellulase EG V has been described for example in the international patent application WO 91/17243. The amount of protein can be measured for example by using the BCA Pierce method as described by R. E. Brown et al. in Anal. Biochem. 1989, vol. 180, p. 136 - 139.

A preparation of an above mentioned Bacillus cellulase available from Kao Corp. under the trade mark KAC® 500 or KAC® 700 may be used as comparison, resulting in general in a very low tensile strength loss as compared to the control washing experiment with no cellulase present.

The attack of cellulases on protruding microfibrils, pills and cotton fluff on the surface of a cotton fabric results in an optically visible removal of that pills. To test the effect, washings are to be performed using a detergent with and without cellulase, as described for the determination of TSL. The antipilling effect, too, can best be seen after an increasing number of wash cycles. Therefore a number of 15 to 40 wash cycles are generally used to demonstrate this effect of cellulases.

There are three different methods that can be used for quantification of this effect:
1. visual evaluation by a test group (panel)
2. measurement of light reflection (L-value of the CIELAB-system)
3. determination of the cotton fluffs by means of optical measurement

The determination using the L-value of the CIELAB-system [Commission Internationale de l'Éclairage] was described by U. Hotz in Tenside Surf. Det. 1993, vol. 30, page 388.

The optical measurement system, which is used in the preferred method of determining the antpilling properties, usually consists of a light source, a microscope tube and a CCD colour camera recording the light reflected from the surface of a fabric. Depending on the amount of pills and fluff on the surface of the fabric the amount of reflected light as measured by digital image analysis changes. Such a system can be used to measure quantitatively the amount of pill and fluff on fabrics, normally after 15 to 40 wash cycles depending on the type and activity of the cellulase added to the detergent. An optical system which can be used to measure the degree of pilling has been described by T. Muller-Kirschbaum and H. Grundmann in SÖFW, vol. 118 (1992), p. 483- 499.

Whatever method is used to determine the antipilling effect of the cellulase to be tested, the standard cellulase EG V has to be tested under the same conditions and it's effect has to be determined by the same method, taking into account the value resulting from the use of the detergent without cellulase. The value obtained for EG V is taken as AP = 100 %.

As can be seen from this definition, the known cellulase EG V from Humicola insolens has a ratio of TSL to AP of 1. As the above mentioned Bacillus cellulase available from Kao Corp. under the trade mark KAC® 500 or KAC® 700 has a low AP and a very low TSL, it can be seen that also the ratio for this cellulase is approximately 1. Cellulases which - according to the invention - may be choosen for use in aqueous laundry solutions, have a ratio of TSL to AP as much as possible below 1, preferably below 0.8 and more particularly in the range of 0.001 to 0.5. A ratio of TSL to AP of for example 0.5 means that only 50% of tensile strength loss is seen at an enzyme concentration yielding the same antipilling effect as the standard cellulase.

The aqueous laundry solution suitably comprises cellulase according to the definition given above in concentrations of 0.01 mg/l to 0.2 mg/l, more particularly 0.015 mg/l to 0.1 mg/l. These concentrations refer to the weight of cellulolytic protein. In addition all ingredients normally found in laundry solutions can be present.

We found that the use of a single cellulase provided by the invention, unlike previously known mixtures of cellulases which provide colour clarification, does not degrade cotton to an undesirable level causing tensile strength loss.

It is further found that in using a cellulase of the definition according to the invention, unlike previously known cellulases which provide colour clarification, the enzyme does not accumulate on the fabric after repeated laundry washing.

As noted before, the present invention generally relates to providing novel cellulases. However, prior to disclosing this invention in more detail, the following terms will be defined:
"Cellulase" is a generic name for enzymes acting on cellulose and its derivatives, and hydrolysing them into glucose, cellobiose or cellooligosaccharides.
The term "single" cellulase used herein is intended to mean a cellulase which is produced by one gene.
"Obtainable from" an organism in connection with a cellulase means that such cellulase has an amino acid sequence which corresponds to the amino acid sequence of a cellulase which may be obtained from that organism.
"Derivative" is intended to indicate a protein which is derived from the native protein by addition of one or more amino acids to either or both the C- and N- terminal end of the native protein, substitution of one or more amino acids at one or a number of different sites in the native amino acid sequence, deletion of one or more amino acids at either or both ends of the native protein or at one or more sites in the amino acid sequence, or insertion of one or more amino acids at one or more sites in the native amino acid sequence. The preparation of a derivative is usually achieved by modifying a DNA sequence which encodes for the native protein, transformation of that DNA sequence into a suitable host and expression of the modified DNA sequence to form the derivative protein. The derivative of the invention includes peptides comprising altered amino acid sequences in comparison with a precursor enzyme amino acid sequence (e.g., a wild type or native state enzyme according to the present invention) and which peptides retain a characteristic enzyme nature of the precursor enzyme but which have altered properties in some specific aspect. For example, an altered cellulase may have an increased pH optimum or increased temperature resistance but will retain its characteristic cellulase activity. Derivatives also includes chemical modifications of amino acid residues within the enzyme molecule.
"Host cell" means a cell which has the capacity to act as a host and expression vehicle for a recombinant DNA vector comprising DNA which encodes for the native protein or a derivative.
The term "cleaning" means the removal of dirt attached to laundry.
The term "pilling" in this respect is the formation of pills and fuzz on the surface of cotton containing fabrics due to broken or disordered fibres.
The term "antipilling" is used to describe the prevention of the formation of pills and fuzz on the surface of cotton containing fabrics as well as the removal of pills and fuzz from cotton containing fabrics. Antipilling normally results in colour clarification when coloured cotton containing fabrics are treated.
The term "colour clarification" in this respect is the reestablishment of the attractive fresh look of coloured fabrics containing or consisting of cellulose based fibres, which have developed a greyish appearance by treatment, especially with laundry detergents, of the coloured fabric.
The term "redeposition" in this respect is deposition of dirt or colour components that were removed from these textiles or fabrics during a laundry washing or textile treatment.
The term "antiredeposition" in this respect is the action of cellulase to prevent or diminish the redeposition of dirt and colour components on the fabric.
By a "laundry solution" is meant an aqueous solution used for washing, rinsing or conditioning, e.g. softening, fabrics.

The present invention relates to choosing a cellulase which is obtainable from microorganisms. As an successful example can be seen those that are deposited according to the Budapest Treaty on the International Recognition of the Deposits of Microorganisms for the Purposes of Patent Procedures, at the Centraal Bureau voor Schimmelcultures, Baarn, The Netherlands on December 23, 1993 under deposition numbers CBS 669.93 and CBS 670.93 (described in international patent application WO-A-95/18219). These strains have been classified as new species of the genus Bacillus, which do not belong to any of the presently known rRNA-groups of Bacillus. As used herein, the deposited species will be referred to as CBS 669.93 and CBS 670.93.

The microorganisms may be obtained for example from water and soil samples collected in alkaline environments such as alkaline soils and soda lakes.

The microorganisms have subsequently been screened using a carboxymethyl cellulose (CMC)-agar diffusion assay. Strains which showed a clearing zone in this test were isolated as potential cellulase producing strains. Genomic gene libraries of the alkali tolerant cellulase producing strains were constructed. Recombinant clones were screened by agar diffusion on CMC-agar. Recombinant clones that showed clearing zones around the colony were isolated. Single cellulases were produced by fermentation of the recombinant clones in 4*YEP-medium for 48 hours at 30°C. The obtained single cellulases, optionally purified as described in Example 1, were tested in the tests defined above to measure TSL and AP.

Surprisingly it was found that the cellulases obtainable from CBS 670.93 or CBS 669.93 show a good performance in both tests and have a ratio of TSL to AP below 1.

In an example for the invention, an approximately 50 kD cellulase (calculated on the basis of the amino acid sequence (SEQ ID No. 2) of the mature protein) derived from CBS 670.93 (referred to as "BCE 103" herein) is described. It has been revealed by analyzing the gene encoding the amino acid sequence of the approximately 50 kD cellulase that this cellulase is 89% identical in sequence and 92.5% similar in sequence to the cellulase CelA of *Bacillus sp.* N-4 (Fukumori et al., J. Bacter., vol. 168, pp. 479-485) by using the TFastA program (Sequence Analysis Software Package 6.0 of Genetic Computer Group. University of Wisconsin, Biotechnology Center, Madison, Wisconsin) as described by Pearson and Lipman in Proc. Nat. Acad. Sci., vol. 85, pp. 2444-2448 (1988).

In another example for the invention, an approximately 63 kD cellulase (calculated on the basis of amino acid sequence of the mature protein) derived from CBS 669.93 (referred to herein as "BCE 113") is described. It has been revealed by analyzing the gene encoding the amino acid sequence of the approximately 63 kD cellulase that this cellulase is 58% identical in sequence and 72% similar in sequence to the cellulase CelB of Bacillus lautus (Jorgensen et al, Gene, vol. 93 (1990), p. 55-60) by using the TFastA program (Sequence Analysis Software Package 6.0 of Genetic Computer Group, University of Wisconsin, Biotechnology Center, Madison, Wisconsin) as described by Pearson and Lipman in Proc. Nat. Acad. Sci., vol. 85 (1990), p. 2444-2448. The amino acid sequence of BCE 113 is given in SEQ ID No. 4.

A cellulase which may be useful for detergents in addition to having a ratio of TSL to AP below 1 usually performs well in the Antiredeposition Test as described in Example 5. Whiteness maintenance of white fabric is measured by a reflectance measurement. The higher the reflectance value, the more effective the tested cellulase is in antiredeposition performance. They also perform well in the Softening Test as described in Example 5. Depilling is the removal of fibrils and/or microfibers that are disordered and/or broken which usually make a coloured cotton containing fabric look greyish. The more disordered and/or broken fibrils are removed the better the coloured cotton containing fabrics look. Depilling effectiveness can be judged by panels or can be quantified by an image analysis system, as specified above for the measurement of AP. Cellulases which fulfil the requirement of the ratio defined above usually exhibit the following properties: They show a delta REM of at least 4 units, preferably at least 5 units, in the Anti Redeposition Test as defined in the Examples, and they show a depilling result which is at least comparable to that of the cellulase obtainable from CBS 670.93.

The cellulases provided by the present invention may be produced by a process which can be developed using genetic engineering. As a first step the gene encoding the cellulase of the present invention can be cloned using λ-phage (expression) vectors and E. coli host cells. Alternatively PCR cloning using consensus primers designed on conserved domains may be used. Expression of the gene encoding the cellulase of the present invention in E. coli is shown to give an active protein.

After a first cloning step in E. coli, a cellulase gene can be transferred to a more preferred industrial expression host such as Bacillus or Streptomyces species, a filamentous fungus such as Aspergillus, or a yeast. High level expression and secretion obtainable in these host organisms allows accumulation of the cellulase of the invention in the fermentation medium from which they can subsequently be recovered.

Cellulases provided by the invention may preferably be used in amounts of 8·10⁻⁵ % by weight (0.8 ppm) to 8·10⁻³ % by weight (80 ppm), more particularly 1·10⁻⁴% by weight (1 ppm) to 4·10⁻³% by weight (40 ppm), referring to the cellulolytic protein, in detergents. Detergent compositions comprising a cellulase provided by the invention may additionally comprise surfactants which may be of the anionic, non-ionic, cationic, amphoteric or zwitterionic type as well as mixtures of these surfactant classes. Such detergent compositions may contain other detergent ingredients known in the art, as e.g. builders, bleaching agents, bleach activators, anti-corrosion agents, sequestering agents, soil release polymers, perfumes, other enzymes, enzyme stabilizers, etc.

Suitable builders are in particular those from the classes of polycarboxylic acids, more particularly polymeric acrylic acids, methacrylic acids, maleic acids, copolymers thereof and oxidized carbohydrates, as described in international patent application WO-A-93/16110, layer silicates, more particularly bentonites, alumosilicates, more particularly zeolites, crystalline or amorphous alkali metal silicates, more particularly sodium silicate, and alkali metal carbonates, more particularly sodium carbonate. The polycarboxylic acids mentioned are normally used in the form of their alkali metal salts, more particularly in the form of their sodium or potassium safts. The zeolites suitably incorporated are in particular those of the A, P or X type or mixtures thereof. Suitable alkali metal silicates are those with molar ratios of SiO₂ to alkali metal oxide of 1.5 to 3.0. Builders such as these are suitably present in such detergents in quantities of 20% by weight to 80% by weight.

Nonionic surfactants may be present in the detergents preferably in quantities of not more than 10% by weight and, more preferably, in quantities of 2% by weight to 6% by weight, based on the detergent as a whole. Suitable nonionic surfactants are alkyl polyglycosides containing 10 to 22 carbon atoms in the alkyl component and alkoxylates, particularly ethoxylates and/or propoxylates, of linear or branched C₁₀₋₂₂ and preferably C₁₂₋₁₈ alcohols. The degree of alkoxylation of the alcohols is between 1 and 20 and preferably between 3 and 10. They may be prepared in known manner by reaction of the corresponding alcohols with the corresponding alkylene oxides. The fatty alcohol derivatives are particularly suitable, although their branched-chain isomers, more particularly so-called oxoalcohols, may be used for the production of useable alkoxylates. Accordingly, the ethoxylates of primary alcohols containing linear dodecyl, tetradecyl, hexadecyl or octadecyl radicals and mixtures thereof are particularly useful. In addition, corresponding ethoxylation and/or propoxylation products of alkyl amines, vicinal diols and carboxylic acid amides, which correspond to the alcohols mentioned in regard to the alkyl component, and of alkyl phenols containing 5 to 12 carbon atoms in the alkyl component may also be used.

Suitable anionic surfactants are in particular those of the sulfate or sulfonate type, although other types, such as soaps, long-chain N-acyl sarcosinates, salts of fatty acid cyanamides or salts of ether carboxylic acids, which may be obtained from long-chain alkyl or alkyl phenyl polyglycol ethers and chloroacetic acid, may also be used. The anionic surfactants are preferably used in the form of the sodium salts. Surfactants are preferably present in quantities of 2% by weight to 30% by weight and more preferably in quantities of 5% by weight to 20% by weight.

Particularly suitable surfactants of the sulfate type are the sulfuric acid monoesters of long- chain primary alcohols of natural and synthetic origin containing 10 to 20 carbon atoms, i.e. the sulfuric acid monoesters of fatty alcohols such as, for example, coconut oil fatty alcohols, tallow fatty alcohols, oleyl alcohol, or the C₁₀₋₂₀ oxoalcohols and those of secondary alcohols of the same chain length. The sulfuric acid monoesters of aliphatic primary alcohols, secondary alcohols and alkyl phenols ethoxylated with 1 to 6 mol ethylene oxide are particularly suitable. Sulfated fatty acid alkanolamides and sulfated fatty acid monoglycerides are also suitable.

The sulfonate-type surfactants are primarily the alkylbenzene sulfonates containing C₉₋₁₅ alkyl groups, sulfosuccinic acid monoesters and diesters containing 6 to 22 carbon atoms in the alcohol components and the esters of a-sulfofatty acids, for example the a-sulfonated methyl or ethyl esters of hydrogenated coconut oil, palm kernel oil or tallow fatty acids. Other suitable surfactants of the sulfonate type are the alkane sulfonates obtainable from C₁₂₋₁₈ alkanes by sulfochlorination or sulfoxidation and subsequent hydrolysis or neutralization or by addition of bisulfite onto olefins and also olefin sulfonates, i.e. mixtures of alkene and hydroxyalkane sulfonates and also disulfonates which are obtained, for example, from long-chain monoolefins with a terminal or internal double bond by sulfonation with gaseous sulfur trioxide and subsequent alkaline or acidic hydrolysis of the sulfonation products.

Bleaching agents are suitably selected from the type containing peroxygen, as hydrogen peroxide, alkali perborate, alkali percarbonate, alkali persilicate and/or alkali persuffate. Particularly suitable are sodium perborate monohydrate and sodium percarbonate. Bleaching agents may be present in amounts of 5% by weight to 25% by weight, more particularly 7% by weight to 20% by weight.

Bleach activator compounds include in particular N- or O-acyl compounds, for example polyacylated alkylene diamines, more particularly tetraacetyl ethylene diamine, N-acylated triazines, more particularly 1,5-diacetyl-2,4-dioxohexahydro-1,3,5-triazine, acylated glycolurils, more particularly tetraacetyl glycoluril, N-acylated hydantoins, hydrazides, triazoles, urazoles, diketopiperazines, sulfuryl amides and cyanurates, also carboxylic anhydrides, more particularly phthalic anhydride, carboxylic acid esters, more particularly sodium isononanoyloxy benzene sulfonate, and acylated sugar derivatives, more particularly pentaacetyl glucose. The bleach activator may be coated in the usual way with shell-forming substances or may be granulated, optionally using granulation aids, and if desired may contain other additives, for example dye. A bleach activator which forms peroxocarboxylic acids with 2 to 12 carbon atoms, in particular peroxoacetc acid, under the washing conditions is preferably used. A particularly suitable bleach activator is tetraacetyl ethylene diamine (TAED) granulated with carboxymethyl cellulose with average particle sizes of 0.01 mm to 0.8 mm, which may be produced by the process described in European Patent EP-B-0 037 026. In addition to the above mentioned bleach activators or even subtituting them so-called bleach catalysts may be used, which are transition metal complexes.

Enzymes which may be present in the detergents, in addition to the cellulase according to the definition, are proteases, lipases, cutinases, amylases, pullulanases, other cellulases, hemicellulases, xylanases, oxidases and/or peroxidases. They may be present in amounts up to 5% by weight, suitably 0.2% by weight to 2% by weight.

The detergent compositions may be formulated in any convenient form e.g. as a powder or liquid. For the production of detergents with high apparent density of e.g. 650 g/l to 950 g/l a method using an extrusion step, as described in European patent EP-B-0 486 592, is suitable.

Fabric softening compositions comprising the cellulase provided by the invention may comprise further to this cellulase cationic surfactants, preferably of the so-called esterquat type, which are capable of fabric softening and which may increase the fabric softening properties of the compositions.

### Examples

### Example 1: Production of cellulases

### - Screening for cellulase producing microorganisms

Two methods were applied for the isolation of cellulase-producing microorganisms:
1) the soil and water samples were suspended in 0.85% saline solution and directly used in the carboxymethyl cellulose (CMC)-agar diffusion assay for detection of cellulase producing colonies.
2) The soil and water samples were enriched for cellulase containing strains by incubation in a cellulose containing liquid minimal medium or GAM-medium for 1 to 3 days at 40°C. Cultures that showed bacterial growth were analyzed for cellulase activity using the CMC-agar diffusion assay for detection of cellulase producing colonies.

### - Isolation of alkalitolerant, cellulase producing strains

Strains that showed clearing zones in the agar diffusion assay were fermented in 25 millilitre GAM-medium in 100 millilitre shake flasks in an Incubator Shaker (New Brunswick Scientific, Edison, NJ, USA), at 250 r.p.m. at 40°C for 72 hours. CMCase activity was determined in the culture broth at pH 9 and 40°C.

### - Isolation of cellulase genes

Genomic gene libraries of the alkalitolerant cellulase producing strains were constructed in plasmid pTZ18R (Mead, D.A., et al. (1986) Protein Engineering 1, 67). Recombinant clones were screened by agar diffusion on CMC-agar as described by Wood, P.J., et al. (1988) Methods in Enzymology 160, 59-74. Strains that showed clearing zones around the colony were isolated. The CMCase activity of the recombinant strains was determined after fermentation for 48 hours at 30°C in 4*YEP-medium. The plasmid DNA of the recombinant strains was isolated and the inserts were characterized by restriction enzyme analysis and nucleotide sequence analysis.

### - Media

The minimal medium (pH 9.7) used in the CMC-agar diffusion assay and the enrichment procedure, consisted of KNO₃ 1%, Yeast extract (Difco) 0.1%, KH₂PO₄ 0.1%, MgSO₄·7H₂O 0.02%, Na₂CO₃ 1%, NaCI 4% and 0.25% CMC (Sigma C-4888). For solidification 1.5% agar was added.

The complex medium (GAM) used for enzyme production of the donor strains consisted of Peptone (Difco) 0.5%, Yeast extract (Difco) 0.5%, Glucose·H₂O 1%, KH₂PO₄ 0.1%, MgSO₄·7H₂O 0.02%, Na₂CO₃ 1%, NaCI 4%. The pH was adjusted to 9.5 with 4M HCI after which 1% CMC was added.

The complex medium (4*YEP) used for the enzyme production in E. coli recombinant strains consisted of Yeast extract (Difco) 4%, Peptone (Difco) 8%, lactose 0.2%, 100 µg/ml ampicilline.

### - CMC-agar diffusion assay for colonies

Cell suspensions in 0.85% saline solution were plated on CMC-containing minimal medium. After incubation for 1 to 3 days at 40°C, the plates were replica plated and the parent plate was flooded with 0.1% Congo Red for 15 minutes. The plates were destained with 1M NaCl for 30 minutes. The strains that showed a clearing zone around the colony were isolated as potential cellulases producing microorganisms.

### - CMC-agar diffusion assay for liquid fractions

Aliquots of 40 µl of enzyme solution or fermentation broth were pipetted in wells punched out from a layer of 5 mm of minimal medium in a petri dish. After incubation for 16 hours at 40°C cellulase activity was detected by Congo Red / NaCl treatment. The diameter of the clearing zone is a measure for the CMCase activity.

### - Resulting cellulase

These experiments resulted in the isolation of a cellulase producing microorganism which was deposited thereafter as CBS 670.93. The microorganism was classified as a new species of the genus Bacillus. Cloning experiments with the CBS 670.93 strain as a donor strain resulted in the isolation of an E. coli clone which was able to produce a cellulase called BCE 103. The nucleotide sequence of the gene coding for said cellulase was analysed. From the cellulase BCE 103 the N-terminal amino acid sequence was determined using standard methods for obtaining and sequencing peptides (Finlay & Geisow (Eds.), Protein Sequencing - a practical approach, 1989, IRL Press). The amino acid sequence of the cellulase was deduced from the nucleotide sequence, using the N-terminal amino acid sequence for the starting point of the mature protein.

The nucleotide sequence for BCE 103 is shown in SEQ ID No. 1 and the amino acid sequence is shown in SEQ ID No. 2.

### - Purification of the cellulase

After the fermentation the cells were separated from the culture liquid by centrifugation (8000 rpm). The cellulase in the supernatant was precipitated with ammonium sulphate (65% saturation). The precipitate was dissolved in 25 mM phosphate buffer pH 7 + 5 mM EDTA until a conductivity of 7 mS/cm. This solution was applied to a Q-Sepharose FF (diameter 5 cm, length 10 cm) Anion Exchange column, after which the column was washed with 25 mM phosphate buffer pH 7 + 5 mM EDTA until an absorbency of 0.2 AU. A gradient of 0 to 0.5 M NaCl in 25 mM phosphate pH 7 was applied to the column in 80 minutes followed by a gradient from 0.5 to 1 M NaCl in 10 minutes. Depending on which cellulase was applied to the column, elution took place in the first or the second gradient. After elution the column was cleaned (upflow) with 1 M NaOH and equilibrated again with 25 mM phosphate pH 7 + 5 mM EDTA. Depending on the elution the obtained cellulase had a purity of up to about 80%.

### - Characterization

### CMC'ase assay

Assays for cellulase activity were performed using modified methods of the PAHBAH method (Lever M. Anal. Biochem. 1972,47, 273-279 and Lever M. Anal. Biochem. 1977, 81, 21-27).

### Procedure:

A test tube is filled with 250 µl 2.5% CMC in 50 mM glycine buffer pH 9 (CMC-low viscosity is purchased from Sigma) and 250 µl aliquots cellulase, diluted in the appropriate buffer. The test tube is incubated for 30 minutes at 40°C in a waterbath, whereafter 1.5 ml of a daily fresh prepared PAHBAH solution (1% PAHBAH in 100 ml 0.5 M NaOH with 100 µl bismuth solution (containing 48.5 g bismuth nitrate, 28.2 g potassium sodium tartrate and 12.0 g NaOH in 100 ml) is added. The mixture is heated at 70°C for 10 minutes, after which it is cooled on ice for 2 minutes. The absorption is measured at 410 nm. To eliminate the background absorbance of the enzyme samples a control experiment is executed as follows: a tube with substrate is incubated under the same conditions as the test tube. After the incubation 1.5 ml PAHBAH and the enzyme preparation is added (in this order). One unit (U) is defined as the amount of enzyme producing 1 µmol of glucose from CMC equivalent determined as reducing sugars per minute per gram product.

The buffer used for the determination of the pH/temperature profiles is a phosphate/citrate system. The pH/temperature profiles were determined using a fixed enzyme concentration which fits in the linear range of the dose response profile measured at pH 7 and 40°C. This enzyme concentration was used for the measurement of the activities under all other determined conditions.

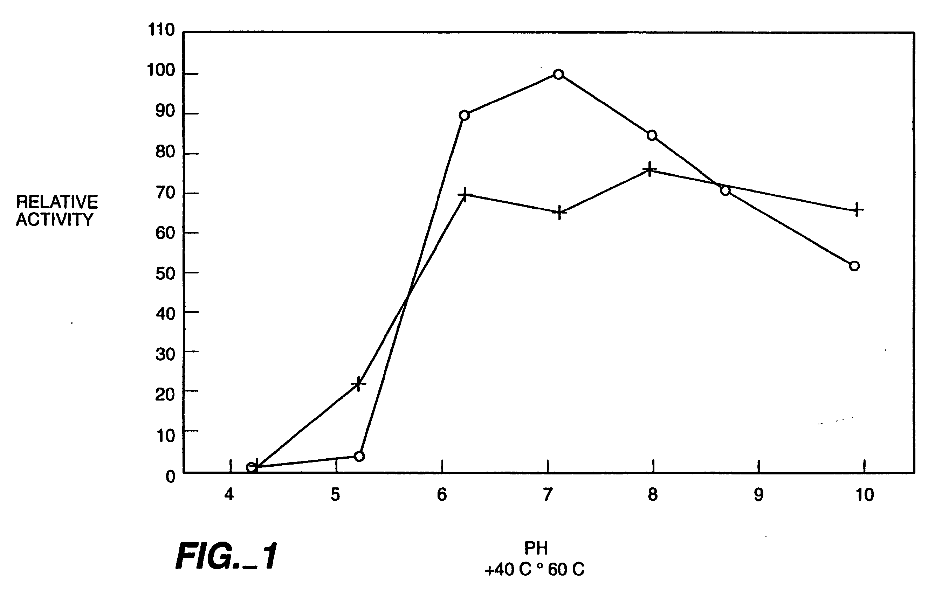

The results for the cellulase BCE 103 are shown in Figure 1. This cellulase shows good activities at alkaline pH, which makes it suitable for application in detergents with an alkaline pH.

### Example 2

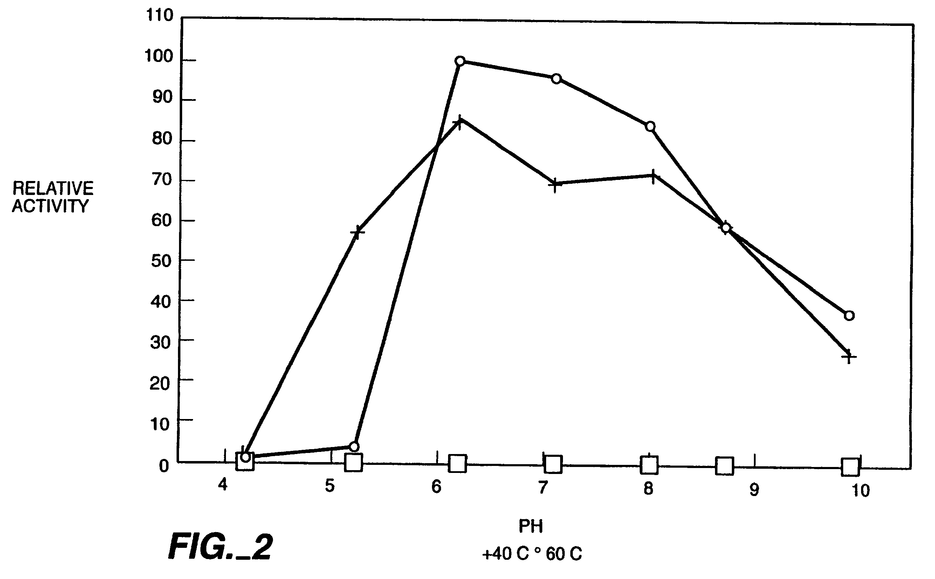

Similar procedures starting with the alkalophilic bacillus strain CBS 669.93 resulted in cellulase BCE 113. The results for this cellulase BCE 113 are shown in Figure 2. This cellulase also shows good activities at alkaline pH, which makes it suitable for application in detergents with an alkaline pH.

### Example 3: Measurement of tensile strength and antipilling

As described for the evaluation of TSL, washing experiments were performed using as detergent matrix a Colour Detergent without bleach, without perfume and enzymes (105 g detergent per wash cycle, pH 10.5), as washing machine a type Miele® W 717, temperature 40°C, program "Normalprogramm", with water of a hardness of 16°dH (German hardness), wash load 3.5 kg, 25 washes.

Experiments using a composition according to the invention (D1) as well as comparisons (C1 to C3) were run in parallel in identical machines:
- C1:: detergent matrix without cellulase
- C2:: detergent matrix + 0.288 mg endoglucanase V from Humicola insolens
- C3:: detergent matrix + cellulase mixture from Humicola insolens sold as granules Celluzyme® 0.7T
- D1:: detergent matrix + 0.288 mg cellulase BCE 103
- D2:: detergent matrix + 0.288 mg cellulase BCE 113

**Table 1: Results of TSL-measurements [%]**

| **Composition** | **TSL** |
|---|---|
| **C1** | 0 |
| **C2** | 100 |
| **C3** | 38 |
| **D1** | 12 |

Using washing machines of type Miele® W 914, under otherwise identical conditions, gave the following results:

**Table 2: Results of TSL-measurements [%]**

| **Composition** | **TSL** |
|---|---|
| **C1** | 0 |
| **C2** | 100 |
| **D2** | 0.6 |

### Example 4: Measurement of antipilling and Calculation of the ratio TSL to AP

The evaluation of antipilling properties was done with increased concentrations of cellulases for better quantitatve evaluation of the effect. A Colour Detergent (5 g/l, 10 wash cycles at 40°C) with the addition of cellulase as given in Table 3 was used on "pilled" sweat shirt cotton material (washed 25 times at 60°C with a detergent without cellulase). Evaluation of the pilling was done with the optical measurement system as described before; a degree of pilling of 0% was assigned to the "pilled" material.

**Table 3: Results of AP-measurements [%]**

| **Enzyme concentration** | **degree of pilling** | | **AP [%]of BCE 103** |
|---|---|---|---|
| | **EG V** | **BCE 103** | |
| **25 µg/ml** | -12.8 % | -8.4 % | 65 % |
| **37,5 µg/ml** | -16.0 % | -9.6 % | 59 % |
| **50 µg/ml** | -22.8 % | -15.6 % | 68 % |

An average AP of 64% can be calculated for BCE 103 cellulase. BCE 113 cellulase showed under the same conditions an average AP of 100%.

Using the values for TSL in Tables 1 and 2, the ratios of TSL to AP for the various cellulases are as in the following Table 4:

**Table 4: Ratio TSL to AP**

| **Enzyme** | **Ratio** |
|---|---|
| **EG V** | 1 |
| **BCE 103** | ≈0.2 |
| **BCE 113** | ≈0.02 |

### Example 5: Further test procedures

### - Anti redeposition test

20 ml 0.5% pigmented soil (fresh prepared, daily and consisting of 86% kaolin, 8% soot (Flammruß 101, obtained from Degussa AG), 4% iron oxide black and 2% iron oxide yellow (from Henkel Genthin GmbH)), in a detergent (Persil color® without enzymes, 5 g/l, pH 8.5) was, under agitating (90 rpm) incubated with white cotton fabric (prewashed, 5 cm diameter, obtained from Windelbleiche, Krefeld). Cellulase was added until a final concentration of 1 mU/ml. The mixture was incubated for 30 minutes at 40°C, 90 rpm. As a control the same incubation was carried out without the addition of cellulase. After the incubation the fabric was rinsed thoroughly with running cold water. After drying the whiteness of the fabric was measured by remission (4 measurements per fabric) using a Micro colour Dr. Lange® Colourimeter. The control value was substracted from the sample value. The results, expressed as delta Rem, are shown in Table 5.

### - Fibre Damage Test

One pad of cotton wool (100% cotton, Warenhandels GmbH, Buchholz, Marke Olivia, Selling agency: Aldi) was incubated in 40 ml wash liquor (Persil color® without enzyme, 5 g/l pH 8.5), cellulase at a final concentration of 1 mU/ml was added in a sealed flask and incubated for 20 hours at 40°C under agitation (90 rpm). After the incubation, fibre damage was monitored by the measurement of the quantity of the reducing sugars in solution, using the PAHBAH method described in Example 1. As a control the same incubation was carried out without the addition of cellulase. The results are shown in Table 5.

### - Adsorption Test

White cotton fabric (Windelbleiche, Bielefeld) prewashed with german Persil® without enzymes at 60°C, was cut round to 9 cm diameter (approx. 0.920 gram). One cotton swatch was incubated in 50 ml 50 mM glycine-NaOH buffer pH 9 including 0.1% SDS and 1 ml cellulase sample (600 mU/ml) for 60 minutes at 30°C. 2 ml samples were taken at T=0 and at T=60 minutes and were diluted directly (1:2) with 50 mM MES-buffer pH 6.5 and stored at 4°C until measurement. As control the same incubation was carried out without the addition of cotton textile. The activity measurement was determined with a PAHBAH method as described in Example 3, but at pH 6.5 in 50 mM MES buffer. The adsorption was expressed as relative adsorption where the activity applied at the start of the experiment was set as 100%, T=0. 100% activity value - remaining activity (%) = adsorption (%). The results are shown in Table 5.

**Table 5: Results of the Antiredeposition Test, Fibre Damage Test and Adsorption Test**

| **Enzyme** | **Antiredeposition [delta REM]** | **Fibre Damage [mU]** | **Adsorption [%]** |
|---|---|---|---|
| **BCE 103** | 5.0 | 0.025 | 7 |
| **KAC®** ^{**a)**} | 7.5 | 0.006 | 0 |
| **EG V** | 1.2 | 0.155 | 36 |

| | | | |
|---|---|---|---|
| a): Cellulase of Kao Corporation | | | |

Cellulase BCE 113 performed in these tests at least as well as cellulase BCE 103.

### - Softening test

The softness of fabrics treated as in Example 3, but after 15 wash cycles, was rated by an expert panel (5 persons) who awarded grades between 0 (fabric washed 25 times with a detergent without cellulase) and 6 (fabric prior to any wash) by the feel of the fabrics. Compositions as defined in Example 2 were used in the washings. The average rates are given in Table 6. It can be seen that the composibons according to the invention showed the best performance.

**Table 6: Results of the Softening Test**

| **Composition** | **Rate** |
|---|---|
| **C1** | 0 |
| **C2** | 2.1 |
| **C3** | 1.5 |
| **D1** | 2.3 |
| **D2** | 2.2 |

### Legend to the figures

Figure 1 shows the relative activities of the cellulase BCE 103. In Example 1 this figure is referred to as the pH/temperature profiles. All activities for both 40 and 60°C are related to the highest activity which is fixed on 100%.
Figure 2 shows the relative activities of the cellulase BCE 113.

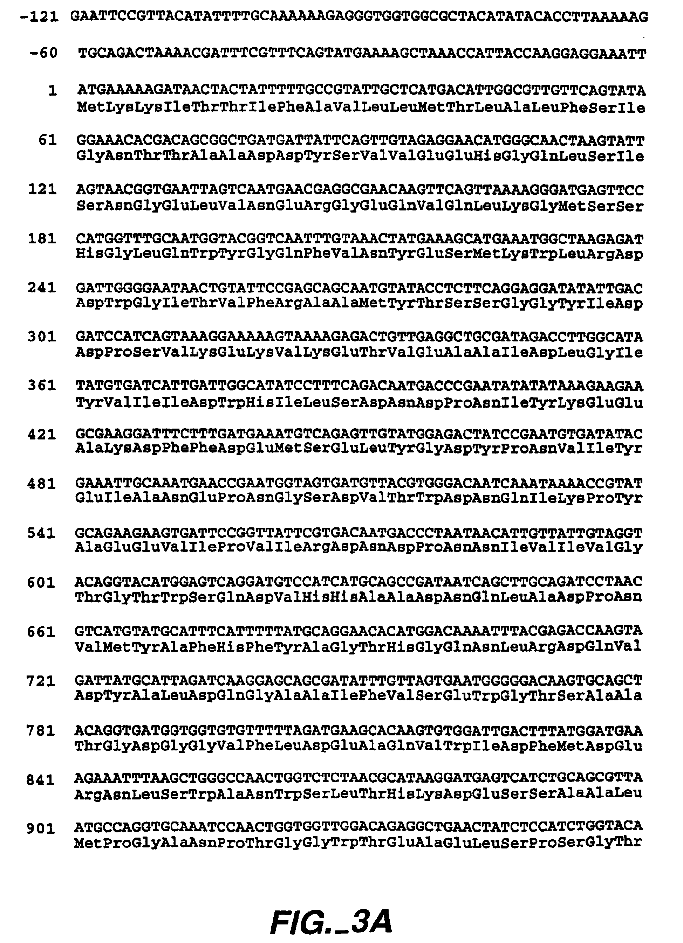

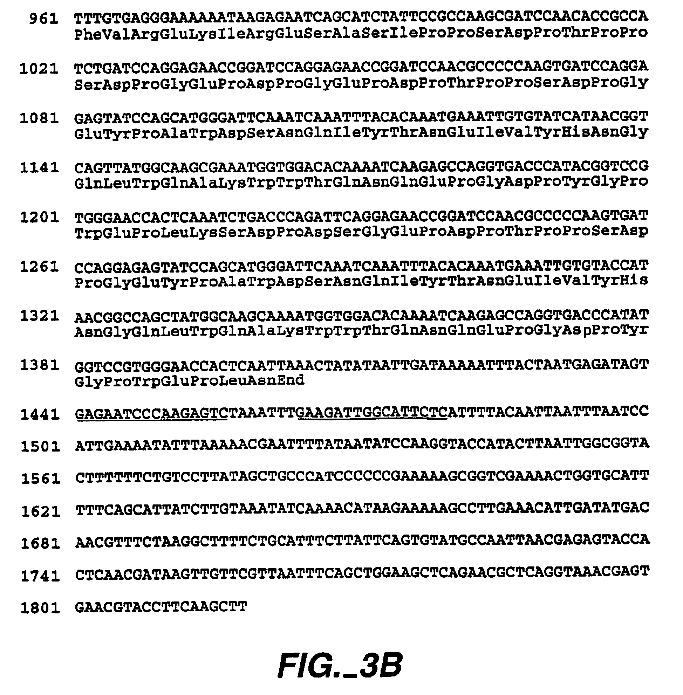

Figure 3 shows the DNA sequence (SEQ ID No. 1) and deduced amino acid sequence (SEQ ID No. 2) of the 50 kD cellulase derived from CBS 670.93 with the leader peptide sequence shaded, which upon secretion is cleaved to yield the mature enzyme.

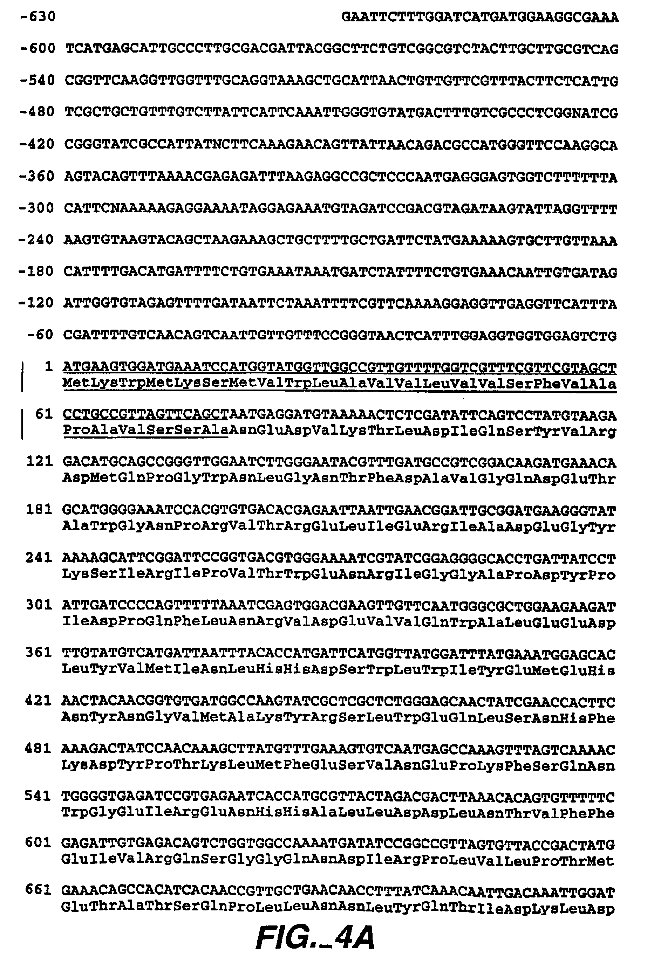

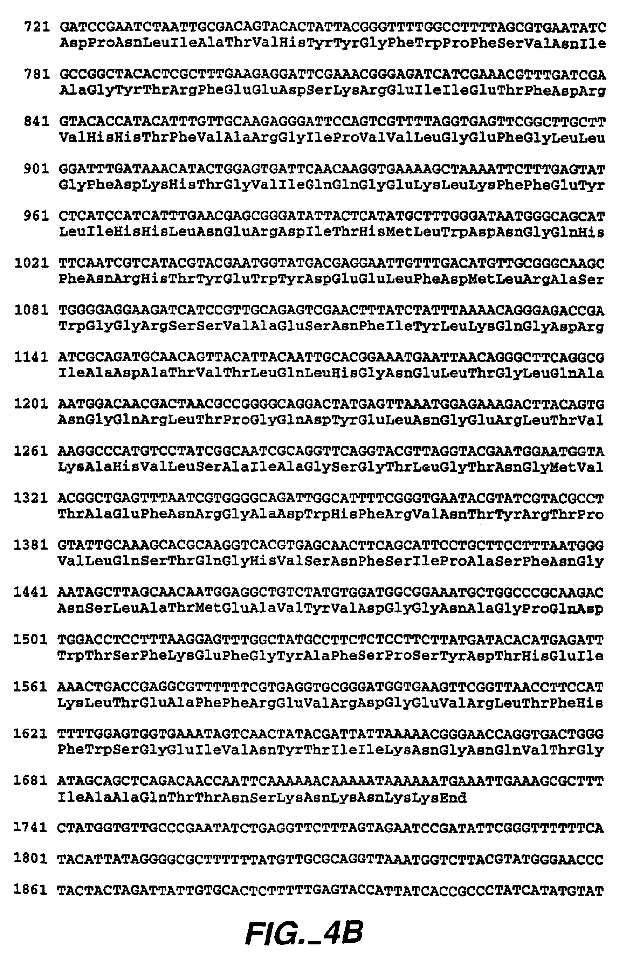

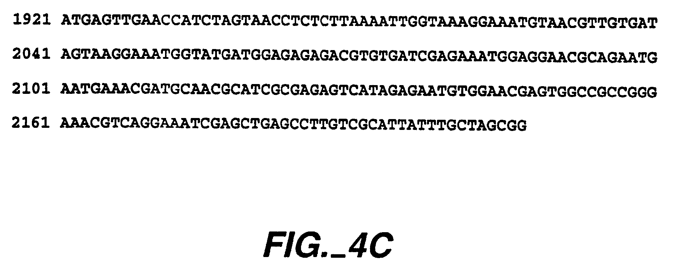

Figure 4 shows the DNA sequence (SEQ ID No. 3) and deduced amino acid sequence (SEQ ID No. 4) of the 63 kD cellulase derived from CBS 669.93 with the leader peptide sequence underlined, which upon secretion is cleaved to yield the mature enzyme.

## Claims

1. Method to provide single cellulases for detergent applications, **characterized by** the steps of
(a) isolation of alkalitolerant, cellulase producing microorganisms that can be obtained from water or soil samples from an alkaline environment,
(b) purification of the cellulases from these strains,
(c) determination of tensile strength loss (TSL) during the washing process,
(d) determination of the antipilling properties (AP) during the washing process and
(e) choosing the cellulases with a ratio of tensile strength loss (TSL) to antipilling properties (AP) below 1.

2. Method according to claim 1, starting with a water sample from an alkaline environment.

3. Method according to claim 1, starting with a soil sample from an alkaline environment.

4. Method according to one of claims 1 to 3, with the samples enriched for cellulase containing strains by incubation in a cellulose containing liquid minimal medium or GAM-medium for 1 to 3 days at 40°C.

5. Method according to one of claims 1 to 4, choosing the cellulases with a ratio of tensile strength loss (TSL) to antipilling properties (AP) below 0.8.

6. Method according to claim 5, choosing the cellulases with a ratio of tensile strength loss (TSL) to antipilling properties (AP) in the range from 0.001 to 0.5.

## Patentansprüche

1. Verfahren zum Bilden einzelner Cellulasen für Waschmittel-Anwendungen, **gekennzeichnet durch** folgende Schritte:
(a) Isolieren von alkalitoleranten, Cellulase bildenden Mikroorganismen, die von Wasser- oder Bodenproben aus einer alkalinen Umgebung erhalten werden können,
(b) Reinigen der Cellulasen von diesen Stämmen,
(c) Bestimmen des Zugfestigkeitsverlusts (TSL) während des Waschverfahrens,
(d) Bestimmen der Antipilling-Eigenschaften (AP) während des Waschverfahrens, und
(e) Auswählen der Cellulasen mit einem Verhältnis zwischen dem Zugfestigkeitsverlust (TSL) und den Antipilling-Eigenschaften (AP) von unter 1.

2. Verfahren nach Anspruch 1, beginnend mit einer Wasserprobe aus einer alkalinen Umgebung.

3. Verfahren nach Anspruch 1, beginnend mit einer Bodenprobe aus einer alkalinen Umgebung.

4. Verfahren nach irgendeinem der Ansprüche 1 bis 3 mit den Proben, die für Cellulase enthaltende Stämme durch Inkubation in einem Cellulose enthaltenden flüssigen Minimalmedium oder GAM-Medium 1 bis 3 Tage bei 40°C angereichert wurden.

5. Verfahren nach irgendeinem der Ansprüche 1 bis 4, wobei die Cellulasen mit einem Verhältnis zwischen dem Zugfestigkeitsverlust (TSL) und den Antipilling-Eigenschaften (AP) von unter 0,8 ausgewählt werden.

6. Verfahren nach Anspruch 5, wobei die Cellulasen mit einem Verhältnis zwischen dem Zugfestigkeitsverlust (TSL) und den Antipilling-Eigenschaften (AP) im Bereich von 0,001 bis 0,5 ausgewählt werden.

## Revendications

1. Procédé pour mettre en oeuvre des cellulases uniques destinées à des applications aux détergents, **caractérisé par** les étapes:
(a) isoler des micro-organismes producteurs de cellulases, tolérant des alcalis, qui peuvent être obtenus à partir d'échantillons d'eaux ou de sols provenant d'un environnement alcalin,
(b) purifier les cellulases à partir de ces souches,
(c) déterminer la perte de résistance à la rupture par traction (TSL) au cours du procédé de lavage,
(d) déterminer les propriétés d'anti-boulochage (AP) au cours du procédé de lavage, et
(e) choisir les cellulases ayant un rapport de perte de résistance à la rupture par traction (TSL) aux propriétés d'anti-boulochage (AP) inférieur à 1.

2. Procédé selon la revendication 1, démarrant avec un échantillon d'eau provenant d'un environnement alcalin.

3. Procédé selon la revendication 1, démarrant avec un échantillon de sol provenant d'un environnement alcalin.

4. Procédé selon l'une quelconque des revendications 1 à 3, les échantillons étant enrichis en souches contenant des cellulases par incubation dans un milieu minimal liquide ou un milieu GAM contenant de la cellulose pendant 1 à 3 jours à 40°C.

5. Procédé selon l'une quelconque des revendications 1 à 4, en choisissant les cellulases ayant un rapport de perte de résistance à la rupture par traction (TSL) aux propriétés d'anti-boulochage (AP) inférieur à 0,8.

6. Procédé selon la revendication 5, en choisissant les cellulases ayant un rapport de perte de résistance à la rupture par traction (TSL) aux propriétés d'anti-boulochage (AP) se situant dans la plage comprise entre 0,001 et 0,5.


### SEQUENZPROTOKOLL

<110> Henkel Kommanditgesellschaft auf Aktien
<120> Detergents comprising cellulases
<130> H 1920-1 EP
<140>
   <141>
<150> EP95201115.3
   <151> 1995-04-28
<150> US614115
   <151> 1996-03-12
<150> PCT/EP96/01755
   <151> 1996-04-26
<150> EP96914993.9
   <151> 1996-04-26
<160> 4
<170> PatentIn Ver. 2.1
<210> 1
   <211> 1404
   <212> DNA
   <213> Bacillus sp. CBS 670.93
<220>
   <221> CDS
   <222> (1)..(1404)
<220>
   <221> sig_peptide
   <222> (1)..(78)
<220>
   <221> mat_peptide
   <222> (79)..(1404)
<400> 1
   atg aaa aag ata act act att ttt gcc gta ttg ctc atg aca ttg gcg    48
   Met Lys Lys Ile Thr Thr Ile Phe Ala Val Leu Leu Met Thr Leu Ala
       -25                 -20                 -15
   ttg ttc agt ata gga aac acg aca gcg gct gat gat tat tca gtt gta    96
   Leu Phe Ser Ile Gly Asn Thr Thr Ala Ala Asp Asp Tyr Ser Val Val
   -10                 -5                  -1   1               5
   gag gaa cat ggg caa cta agt att agt aac ggt gaa tta gtc aat gaa    144
   Glu Glu His Gly Gln Leu Ser Ile Ser Asn Gly Glu Leu Val Asn Glu
                   10                  15                  20
   cga ggc gaa caa gtt cag tta aaa ggg atg agt tcc cat ggt ttg caa    192
   Arg Gly Glu Gln Val Gln Leu Lys Gly Met Ser Ser His Gly Leu Gln
           25                  30                  35
   tgg tac ggt caa ttt gta aac tat gaa agc atg aaa tgg cta aga gat   240
   Trp Tyr Gly Gln Phe Val Asn Tyr Glu Ser Met Lys Trp Leu Arg Asp
       40                  45                  50
   gat tgg gga ata act gta ttc cga gca gca atg tat acc tct tca gga   288
   Asp Trp Gly Ile Thr Val Phe Arg Ala Ala Met Tyr Thr Ser Ser Gly
   55                  60                  65                  70
   gga tat att gac gat cca tca gta aag gaa aaa gta aaa gag act gtt   336
   Gly Tyr Ile Asp Asp Pro Ser Val Lys Glu Lys Val Lys Glu Thr Val
                   75                  80                  85
   gag gct gcg ata gac ctt ggc ata tat gtg atc att gat tgg cat atc   384
   Glu Ala Ala Ile Asp Leu Gly Ile Tyr Val Ile Ile Asp Trp His Ile
               90                  95                  100
   ctt tca gac aat gac ccg aat ata tat aaa gaa gaa gcg aag gat ttc   432
   Leu Ser Asp Asn Asp Pro Asn Ile Tyr Lys Glu Glu Ala Lys Asp Phe
           105                 110                 115
   ttt gat gaa atg tca gag ttg tat gga gac tat ccg aat gtg ata tac   480
   Phe Asp Glu Met Ser Glu Leu Tyr Gly Asp Tyr Pro Asn Val Ile Tyr
       120                 125                 130
   gaa att gca aat gaa ccg aat ggt agt gat gtt acg tgg gac aat caa   528
   Glu Ile Ala Asn Glu Pro Asn Gly Ser Asp Val Thr Trp Asp Asn Gln
   135                 140                 145                 150
   ata aaa ccg tat gca gaa gaa gtg att ccg gtt att cgt gac aat gac   576
   Ile Lys Pro Tyr Ala Glu Glu Val Ile Pro Val Ile Arg Asp Asn Asp
                   155                 160                 165
   cct aat aac att gtt att gta ggt aca ggt aca tgg agt cag gat gtc   624
   Pro Asn Asn Ile Val Ile Val Gly Thr Gly Thr Trp Ser Gln Asp Val
               170                 175                 180
   cat cat gca gcc gat aat cag ctt gca gat cct aac gtc atg tat gca   672
   His His Ala Ala Asp Asn Gln Leu Ala Asp Pro Asn Val Met Tyr Ala
           185                 190                 195
   ttt cat ttt tat gca gga aca cat gga caa aat tta cga gac caa gta   720
   Phe His Phe Tyr Ala Gly Thr His Gly Gln Asn Leu Arg Asp Gln Val
       200                 205                 210
   gat tat gca tta gat caa gga gca gcg ata ttt gtt agt gaa tgg ggg   768
   Asp Tyr Ala Leu Asp Gln Gly Ala Ala Ile Phe Val Ser Glu Trp Gly
   215                 220                 225                 230
   aca agt gca gct aca ggt gat ggt ggt gtg ttt tta gat gaa gca caa   816
   Thr Ser Ala Ala Thr Gly Asp Gly Gly Val Phe Leu Asp Glu Ala Gln
                   235                 240                 245
   gtg tgg att gac ttt atg gat gaa aga aat tta agc tgg gcc aac tgg   864
   Val Trp Ile Asp Phe Met Asp Glu Arg Asn Leu Ser Trp Ala Asn Trp
               250                 255                 260
   tct cta acg cat aag gat gag tca tct gca gcg tta atg cca ggt gca   912
   Ser Leu Thr His Lys Asp Glu Ser Ser Ala Ala Leu Met Pro Gly Ala
           265                 270                     275
   aat cca act ggt ggt tgg aca gag gct gaa cta tct cca tct ggt aca     960
   Asn Pro Thr Gly Gly Trp Thr Glu Ala Glu Leu Ser Pro Ser Gly Thr
       280                 285                 290
   ttt gtg agg gaa aaa ata aga gaa tca gca tct att ccg cca agc gat     1008
   Phe Val Arg Glu Lys Ile Arg Glu Ser Ala Ser Ile Pro Pro Ser Asp
   295                 300                 305                 310
   cca aca ccg cca tct gat cca gga gaa ccg gat cca gga gaa ccg gat     1056
   Pro Thr Pro Pro Ser Asp Pro Gly Glu Pro Asp Pro Gly Glu Pro Asp
                   315                 320                 325
   cca acg ccc cca agt gat cca gga gag tat cca gca tgg gat tca aat     1104
   Pro Thr Pro Pro Ser Asp Pro Gly Glu Tyr Pro Ala Trp Asp Ser Asn
               330                 335                 340
   caa att tac aca aat gaa att gtg tat cat aac ggt cag tta tgg caa     1152
   Gln Ile Tyr Thr Asn Glu Ile Val Tyr His Asn Gly Gln Leu Trp Gln
           345                 350                 355
   gcg aaa tgg tgg aca caa aat caa gag cca ggt gac cca tac ggt ccg     1200
   Ala Lys Trp Trp Thr Gln Asn Gln Glu Pro Gly Asp Pro Tyr Gly Pro
       360                 365                 370
   tgg gaa cca ctc aaa tct gac cca gat tca gga gaa ccg gat cca acg     1248
   Trp Glu Pro Leu Lys Ser Asp Pro Asp Ser Gly Glu Pro Asp Pro Thr
   375                 380                 385                 390
   ccc cca agt gat cca gga gag tat cca gca tgg gat tca aat caa att     1296
   Pro Pro Ser Asp Pro Gly Glu Tyr Pro Ala Trp Asp Ser Asn Gln Ile
                   395                 400                 405
   tac aca aat gaa att gtg tac cat aac ggc cag cta tgg caa gca aaa     1344
   Tyr Thr Asn Glu Ile Val Tyr His Asn Gly Gln Leu Trp Gln Ala Lys
               410                 415                 420
   tgg tgg aca caa aat caa gag cca ggt gac cca tat ggt ccg tgg gaa     1392
   Trp Trp Thr Gln Asn Gln Glu Pro Gly Asp Pro Tyr Gly Pro Trp Glu
           425                 430                 435
   cca ctc aat taa                                                     1404
   Pro Leu Asn
       440
<210> 2
   <211> 467
   <212> PRT
   <213> Bacillus sp. CBS 670.93
<400> 2
   Met Lys Lys Ile Thr Thr Ile Phe Ala Val Leu Leu Met Thr Leu Ala
    1               5                  10                  15
   Leu Phe Ser Ile Gly Asn Thr Thr Ala Ala Asp Asp Tyr Ser Val Val
               20                  25                  30
   Glu Glu His Gly Gln Leu Ser Ile Ser Asn Gly Glu Leu Val Asn Glu
               35                      40                      45
   Arg Gly Glu Gln Val Gln Leu Lys Gly Met Ser Ser His Gly Leu Gln
       50                      55                      60
   Trp Tyr Gly Gln Phe Val Asn Tyr Glu Ser Met Lys Trp Leu Arg Asp
   65                      70                      75                      80
   Asp Trp Gly Ile Thr Val Phe Arg Ala Ala Met Tyr Thr Ser Ser Gly
                   85                      90                      95
   Gly Tyr Ile Asp Asp Pro Ser Val Lys Glu Lys Val Lys Glu Thr Val
               100                     105                     110
   Glu Ala Ala Ile Asp Leu Gly Ile Tyr Val Ile Ile Asp Trp His Ile
           115                     120                     125
   Leu Ser Asp Asn Asp Pro Asn Ile Tyr Lys Glu Glu Ala Lys Asp Phe
       130                     135                     140
   Phe Asp Glu Met Ser Glu Leu Tyr Gly Asp Tyr Pro Asn Val Ile Tyr
   145                     150                     155                     160
   Glu Ile Ala Asn Glu Pro Asn Gly Ser Asp Val Thr Trp Asp Asn Gln
                   165                     170                     175
   Ile Lys Pro Tyr Ala Glu Glu Val Ile Pro Val Ile Arg Asp Asn Asp
               180                     185                     190
   Pro Asn Asn Ile Val Ile Val Gly Thr Gly Thr Trp Ser Gln Asp Val
           195                     200                     205
   His His Ala Ala Asp Asn Gln Leu Ala Asp Pro Asn Val Met Tyr Ala
       210                     215                     220
   Phe His Phe Tyr Ala Gly Thr His Gly Gln Asn Leu Arg Asp Gln Val
   225                     230                     235                     240
   Asp Tyr Ala Leu Asp Gln Gly Ala Ala Ile Phe Val Ser Glu Trp Gly
                   245                     250                     255
   Thr Ser Ala Ala Thr Gly Asp Gly Gly Val Phe Leu Asp Glu Ala Gln
               260                     265                     270
   Val Trp Ile Asp Phe Met Asp Glu Arg Asn Leu Ser Trp Ala Asn Trp
           275                     280                     285
   Ser Leu Thr His Lys Asp Glu Ser Ser Ala Ala Leu Met Pro Gly Ala
       290                     295                     300
   Asn Pro Thr Gly Gly Trp Thr Glu Ala Glu Leu Ser Pro Ser Gly Thr
   305                     310                     315                     320
   Phe Val Arg Glu Lys Ile Arg Glu Ser Ala Ser Ile Pro Pro Ser Asp
                   325                     330                     335
   Pro Thr Pro Pro Ser Asp Pro Gly Glu Pro Asp Pro Gly Glu Pro Asp
               340                     345                     350
   Pro Thr Pro Pro Ser Asp Pro Gly Glu Tyr Pro Ala Trp Asp Ser Asn
           355                     360                     365
   Gln Ile Tyr Thr Asn Glu Ile Val Tyr His Asn Gly Gln Leu Trp Gln
       370                     375                     380
   Ala Lys Trp Trp Thr Gln Asn Gln Glu Pro Gly Asp Pro Tyr Gly Pro
   385                     390                     395                     400
   Trp Glu Pro Leu Lys Ser Asp Pro Asp Ser Gly Glu Pro Asp Pro Thr
                   405                     410                     415
   Pro Pro Ser Asp Pro Gly Glu Tyr Pro Ala Trp Asp Ser Asn Gln Ile
               420                     425                     430
   Tyr Thr Asn Glu Ile Val Tyr His Asn Gly Gln Leu Trp Gln Ala Lys
           435                     440                     445
   Trp Trp Thr Gln Asn Gln Glu Pro Gly Asp Pro Tyr Gly Pro Trp Glu
       450                     455                     460
   Pro Leu Asn
   465
<210> 3
   <211> 1725
   <212> DNA
   <213> Bacillus sp. CBS 669.93
<220>
   <221> CDS
   <222> (1)..(1725)
<220>
   <221> sig_peptide
   <222> (1)..(78)
<220>
   <221> mat_peptide
   <222> (79)..(1725)
<400> 3
   atg aag tgg atg aaa tcc atg gta tgg ttg gcc gtt gtt ttg gtc gtt    48
   Met Lys Trp Met Lys Ser Met Val Trp Leu Ala Val Val Leu Val Val
       -25                 -20                 -15
   tcg ttc gta gct cct gcc gtt agt tca gct aat gag gat gta aaa act    96
   Ser Phe Val Ala Pro Ala Val Ser Ser Ala Asn Glu Asp Val Lys Thr
   -10                 -5                  -1   1               5
   ctc gat att cag tcc tat gta aga gac atg cag ccg ggt tgg aat ctt    144
   Leu Asp Ile Gln Ser Tyr Val Arg Asp Met Gln Pro Gly Trp Asn Leu
               10                  15                  20
   ggg aat acg ttt gat gcc gtc gga caa gat gaa aca gca tgg gga aat    192
   Gly Asn Thr Phe Asp Ala Val Gly Gln Asp Glu Thr Ala Trp Gly Asn
           25                  30                  35
   cca cgt gtg aca cga gaa tta att gaa cgg att gcg gat gaa ggg tat    240
   Pro Arg Val Thr Arg Glu Leu Ile Glu Arg Ile Ala Asp Glu Gly Tyr
       40                  45                  50
   aaa agc att cgg att ccg gtg acg tgg gaa aat cgt atc gga ggg gca    288
   Lys Ser Ile Arg Ile Pro Val Thr Trp Glu Asn Arg Ile Gly Gly Ala
   55                  60                  65                  70
   cct gat tat cct att gat ccc cag ttt tta aat cga gtg gac gaa gtt    336
   Pro Asp Tyr Pro Ile Asp Pro Gln Phe Leu Asn Arg Val Asp Glu Val
                   75                  80                  85
   gtt caa tgg gcg ctg gaa gaa gat ttg tat gtc atg att aat tta cac    384
   Val Gln Trp Ala Leu Glu Glu Asp Leu Tyr Val Met Ile Asn Leu His
               90                  95                  100
   cat gat tca tgg tta tgg att tat gaa atg gag cac aac tac aac ggt    432
   His Asp Ser Trp Leu Trp Ile Tyr Glu Met Glu His Asn Tyr Asn Gly
           105                 110                 115
   gtg atg gcc aag tat cgc tcg ctc tgg gag caa cta tcg aac cac ttc    480
   Val Met Ala Lys Tyr Arg Ser Leu Trp Glu Gln Leu Ser Asn His Phe
       120                 125                 130
   aaa gac tat cca aca aag ctt atg ttt gaa agt gtc aat gag cca aag    528
   Lys Asp Tyr Pro Thr Lys Leu Met Phe Glu Ser Val Asn Glu Pro Lys
   135                 140                 145                 150
   ttt agt caa aac tgg ggt gag atc cgt gag aat cac cat gcg tta cta     576
   Phe Ser Gln Asn Trp Gly Glu Ile Arg Glu Asn His His Ala Leu Leu
                   155                 160                 165
   gac gac tta aac aca gtg ttt ttc gag att gtg aga cag tct ggt ggc     624
   Asp Asp Leu Asn Thr Val Phe Phe Glu Ile Val Arg Gln Ser Gly Gly
               170                 175                 180
   caa aat gat atc cgg ccg tta gtg tta ccg act atg gaa aca gcc aca     672
   Gln Asn Asp Ile Arg Pro Leu Val Leu Pro Thr Met Glu Thr Ala Thr
           185                 190                 195
   tca caa ccg ttg ctg aac aac ctt tat caa aca att gac aaa ttg gat     720
   Ser Gln Pro Leu Leu Asn Asn Leu Tyr Gln Thr Ile Asp Lys Leu Asp
       200                 205                 210
   gat ccg aat cta att gcg aca gta cac tat tac ggg ttt tgg cct ttt     768
   Asp Pro Asn Leu Ile Ala Thr Val His Tyr Tyr Gly Phe Trp Pro Phe
   215                 220                 225                 230
   agc gtg aat atc gcc ggc tac act cgc ttt gaa gag gat tcg aaa cgg     816
   Ser Val Asn Ile Ala Gly Tyr Thr Arg Phe Glu Glu Asp Ser Lys Arg
                   235                 240                 245
   gag atc atc gaa acg ttt gat cga gta cac cat aca ttt gtt gca aga     864
   Glu Ile Ile Glu Thr Phe Asp Arg Val His His Thr Phe Val Ala Arg
               250                 255                 260
   ggg att cca gtc gtt tta ggt gag ttc ggc ttg ctt gga ttt gat aaa     912
   Gly Ile Pro Val Val Leu Gly Glu Phe Gly Leu Leu Gly Phe Asp Lys
           265                 270                 275
   cat act gga gtg att caa caa ggt gaa aag cta aaa ttc ttt gag tat     960
   His Thr Gly Val Ile Gln Gln Gly Glu Lys Leu Lys Phe Phe Glu Tyr
       280                 285                 290
   ctc atc cat cat ttg aac gag cgg gat att act cat atg ctt tgg gat     1008
   Leu Ile His His Leu Asn Glu Arg Asp Ile Thr His Met Leu Trp Asp
   295                 300                 305                 310
   aat ggg cag cat ttc aat cgt cat acg tac gaa tgg tat gac gag gaa     1056
   Asn Gly Gln His Phe Asn Arg His Thr Tyr Glu Trp Tyr Asp Glu Glu
                   315                 320                 325
   ttg ttt gac atg ttg cgg gca agc tgg gga gga aga tca tcc gtt gca     1104
   Leu Phe Asp Met Leu Arg Ala Ser Trp Gly Gly Arg Ser Ser Val Ala
               330                 335                 340
   gag tcg aac ttt atc tat tta aaa cag gga gac cga atc gca gat gca     1152
   Glu Ser Asn Phe Ile Tyr Leu Lys Gln Gly Asp Arg Ile Ala Asp Ala
           345                 350                 355
   aca gtt aca tta caa ttg cac gga aat gaa tta aca ggg ctt cag gcg     1200
   Thr Val Thr Leu Gln Leu His Gly Asn Glu Leu Thr Gly Leu Gln Ala
       360                 365                 370
   aat gga caa cga cta acg ccg ggg cag gac tat gag tta aat gga gaa     1248
   Asn Gly Gln Arg Leu Thr Pro Gly Gln Asp Tyr Glu Leu Asn Gly Glu
   375                 380                 385                 390
   aga ctt aca gtg aag gcc cat gtc cta tcg gca atc gca ggt tca ggt     1296
   Arg Leu Thr Val Lys Ala His Val Leu Ser Ala Ile Ala Gly Ser Gly
                   395                 400                 405
   acg tta ggt acg aat gga atg gta acg gct gag ttt aat cgt ggg gca     1344
   Thr Leu Gly Thr Asn Gly Met Val Thr Ala Glu Phe Asn Arg Gly Ala
               410                 415                 420
   gat tgg cat ttt cgg gtg aat acg tat cgt acg cct gta ttg caa agc     1392
   Asp Trp His Phe Arg Val Asn Thr Tyr Arg Thr Pro Val Leu Gln Ser
           425                 430                 435
   acg caa ggt cac gtg agc aac ttc agc att cct gct tcc ttt aat ggg     1440
   Thr Gln Gly His Val Ser Asn Phe Ser Ile Pro Ala Ser Phe Asn Gly
       440                 445                 450
   aat agc tta gca aca atg gag gct gtc tat gtg gat ggc gga aat gct     1488
   Asn Ser Leu Ala Thr Met Glu Ala Val Tyr Val Asp Gly Gly Asn Ala
   455                 460                 465                 470
   ggc ccg caa gac tgg acc tcc ttt aag gag ttt ggc tat gcc ttc tct     1536
   Gly Pro Gln Asp Trp Thr Ser Phe Lys Glu Phe Gly Tyr Ala Phe Ser
                   475                 480                 485
   cct tct tat gat aca cat gag att aaa ctg acc gag gcg ttt ttt cgt     1584
   Pro Ser Tyr Asp Thr His Glu Ile Lys Leu Thr Glu Ala Phe Phe Arg
               490                 495                 500
   gag gtg cgg gat ggt gaa gtt cgg tta acc ttc cat ttt tgg agt ggt     1632
   Glu Val Arg Asp Gly Glu Val Arg Leu Thr Phe His Phe Trp Ser Gly
           505                 510                 515
   gaa ata gtc aac tat acg att att aaa aac ggg aac cag gtg act ggg     1680
   Glu Ile Val Asn Tyr Thr Ile Ile Lys Asn Gly Asn Gln Val Thr Gly
       520                 525                 530
   ata gca gct cag aca acc aat tca aaa aac aaa aat aaa aaa tga         1725
   Ile Ala Ala Gln Thr Thr Asn Ser Lys Asn Lys Asn Lys Lys
   535                 540                 545
<210> 4
   <211> 574
   <212> PRT
   <213> Bacillus sp. CBS 669.93
<400> 4
   Met Lys Trp Met Lys Ser Met Val Trp Leu Ala Val Val Leu Val Val
    1               5                  10                  15
   Ser Phe Val Ala Pro Ala Val Ser Ser Ala Asn Glu Asp Val Lys Thr
               20                  25                  30
   Leu Asp Ile Gln Ser Tyr Val Arg Asp Met Gln Pro Gly Trp Asn Leu
           35                  40                  45
   Gly Asn Thr Phe Asp Ala Val Gly Gln Asp Glu Thr Ala Trp Gly Asn
       50                  55                  60
   Pro Arg Val Thr Arg Glu Leu Ile Glu Arg Ile Ala Asp Glu Gly Tyr
   65                  70                  75                  80
   Lys Ser Ile Arg Ile Pro Val Thr Trp Glu Asn Arg Ile Gly Gly Ala
                       85                  90                  95
   Pro Asp Tyr Pro Ile Asp Pro Gln Phe Leu Asn Arg Val Asp Glu Val
                   100                 105                 110
   Val Gln Trp Ala Leu Glu Glu Asp Leu Tyr Val Met Ile Asn Leu His
           115                 120                 125
   His Asp Ser Trp Leu Trp Ile Tyr Glu Met Glu His Asn Tyr Asn Gly
       130                 135                 140
   Val Met Ala Lys Tyr Arg Ser Leu Trp Glu Gln Leu Ser Asn His Phe
   145                 150                 155                 160
   Lys Asp Tyr Pro Thr Lys Leu Met Phe Glu Ser Val Asn Glu Pro Lys
                   165                 170                 175
   Phe Ser Gln Asn Trp Gly Glu Ile Arg Glu Asn His His Ala Leu Leu
               180                 185                 190
   Asp Asp Leu Asn Thr Val Phe Phe Glu Ile Val Arg Gln Ser Gly Gly
           195                 200                 205
   Gln Asn Asp Ile Arg Pro Leu Val Leu Pro Thr Met Glu Thr Ala Thr
       210                 215                 220
   Ser Gln Pro Leu Leu Asn Asn Leu Tyr Gln Thr Ile Asp Lys Leu Asp
   225                 230                 235                 240
   Asp Pro Asn Leu Ile Ala Thr Val His Tyr Tyr Gly Phe Trp Pro Phe
                   245                 250                 255
   Ser Val Asn Ile Ala Gly Tyr Thr Arg Phe Glu Glu Asp Ser Lys Arg
               260                 265                 270
   Glu Ile Ile Glu Thr Phe Asp Arg Val His His Thr Phe Val Ala Arg
           275                 280                 285
   Gly Ile Pro Val Val Leu Gly Glu Phe Gly Leu Leu Gly Phe Asp Lys
       290                 295                 300
   His Thr Gly Val Ile Gln Gln Gly Glu Lys Leu Lys Phe Phe Glu Tyr
   305                 310                 315                 320
   Leu Ile His His Leu Asn Glu Arg Asp Ile Thr His Met Leu Trp Asp
                   325                 330                 335
   Asn Gly Gln His Phe Asn Arg His Thr Tyr Glu Trp Tyr Asp Glu Glu
               340                 345                 350
   Leu Phe Asp Met Leu Arg Ala Ser Trp Gly Gly Arg Ser Ser Val Ala
           355                 360                 365
   Glu Ser Asn Phe Ile Tyr Leu Lys Gln Gly Asp Arg Ile Ala Asp Ala
       370                 375                 380
   Thr Val Thr Leu Gln Leu His Gly Asn Glu Leu Thr Gly Leu Gln Ala
   385                 390                 395                 400
   Asn Gly Gln Arg Leu Thr Pro Gly Gln Asp Tyr Glu Leu Asn Gly Glu
                   405                 410                 415
   Arg Leu Thr Val Lys Ala His Val Leu Ser Ala Ile Ala Gly Ser Gly
               420                 425                 430
   Thr Leu Gly Thr Asn Gly Met Val Thr Ala Glu Phe Asn Arg Gly Ala
           435                 440                 445
   Asp Trp His Phe Arg Val Asn Thr Tyr Arg Thr Pro Val Leu Gln Ser
       450                 455                 460
   Thr Gln Gly His Val Ser Asn Phe Ser Ile Pro Ala Ser Phe Asn Gly
   465                 470                 475                 480
   Asn Ser Leu Ala Thr Met Glu Ala Val Tyr Val Asp Gly Gly Asn Ala
                   485                 490                 495
   Gly Pro Gln Asp Trp Thr Ser Phe Lys Glu Phe Gly Tyr Ala Phe Ser
               500                 505                 510
   Pro Ser Tyr Asp Thr His Glu Ile Lys Leu Thr Glu Ala Phe Phe Arg
           515                 520                 525
   Glu Val Arg Asp Gly Glu Val Arg Leu Thr Phe His Phe Trp Ser Gly
       530                 535                 540
   Glu Ile Val Asn Tyr Thr Ile Ile Lys Asn Gly Asn Gln Val Thr Gly
   545                 550                 555                 560
   Ile Ala Ala Gln Thr Thr Asn Ser Lys Asn Lys Asn Lys Lys
                   565                 570